# EUROPÄISCHE PATENTANMELDUNG

(11) **EP 0 838 468 A2**
(43) Veröffentlichungstag der Anmeldung: **29.04.1998**
(21) Anmeldenummer: 97114900.0
(22) Anmeldetag: 28.08.1997
(51) Int. Cl.: C07H 15/04

(54) **Verfahren zur Herstellung von Alpha-D-Glucopyranosido-1,6-mannit und -sorbit aus Alpha-D-Glucopyranosido-1,6-fructose**

(30) Priorität: 10.09.1996 DE 19636625
(71) Anmelder: BAYER AG, 51368 Leverkusen (DE)
(72) Erfinder: Darsow, Gerhard, Dr., 47804 Krefeld (DE)

(57) **Zusammenfassung**

Die als Titelverbindungen genannten Zuckeralkohole können aus dem korrespondierenden Zucker in äquimolaren Mengen durch katalytische Hydrierung in wäßriger Lösung mit Wasserstoff hergestellt werden, wobei man die Hydrierung kontinuierlich bei einem H₂-Druck von 100 bis 400 bar und einer Reaktionstemperatur von 20 bis 80°C an im Festbett angeordneten trägerfreien Formkörpern aus verpreßten Pulvern von Legierungen der Elemente der Eisenuntergruppe der VIII. Nebengruppe des Periodensystem mit Elementen der IV. und V. Nebengruppe durchführt. Die Formkörper besitzen eine Druckfestigkeit von 20 bis 220 N und eine innere Oberfläche von 10 bis 95 m²/g.

## Beschreibung

Die Erfindung betrifft ein kostengünstiges Verfahren zur Herstellung eines äquimolaren Gemisches der diastereomeren Zuckeralkohole α-D-Glucopyranosido-1,6-mannit und α-D-Glucopyranosido-1,6-sorbit aus α-D-Glucopyranosido-1,6-fructose durch kontinuierliche katalytische Hydrierung mit Wasserstoff.

Der Reaktionsverlauf läßt sich durch das folgende Reaktionsschema veranschaulichen:

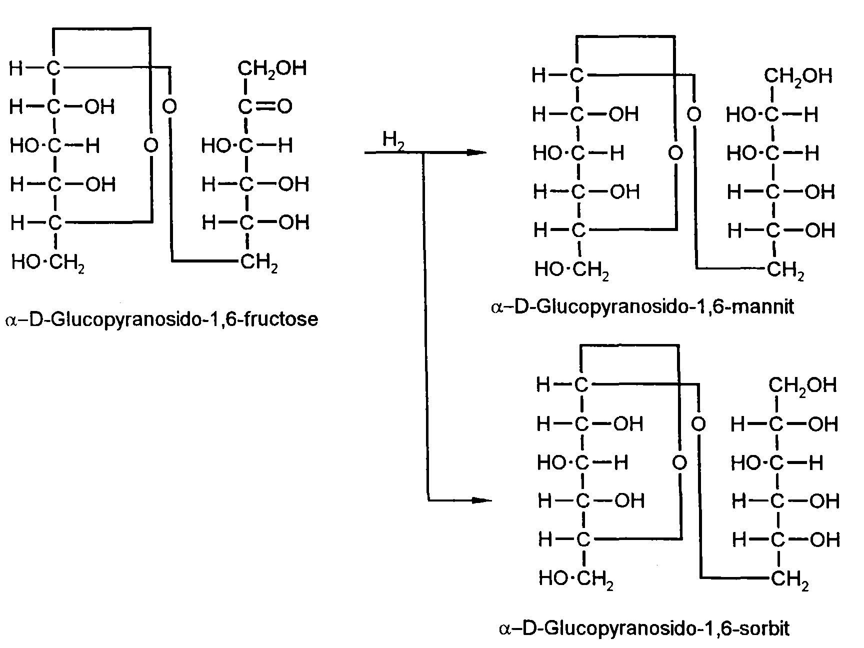

Bei den bekannten Verfahren zur Herstellung von α-D-Glucopyranosido-1,6-sorbit (DE-PS 2 217 628) und α-D-Glucopyranosido-1,6-mannit (DE-AS 2 520 173) wird im diskontinuierlichen Suspensionsverfahren (Batch-process) als Hydrierkatalysator jeweils ein pulverförmiger Nickelkatalysator eingesetzt. Diskontinuierliche Verfahren haben den Nachteil, daß ihre Kapazität relativ zum Reaktionsvolumen sehr klein ist und somit ein Bedarf nach großvolumigen teuren Reaktionsapparaturen und Lagertanks besteht. Energieverbrauch und Personalbedarf sind bei diskontinuierlichen Prozessen verhältnismäßig hoch. Kontinuierliche Pulverkatalysatorverfahren, die mit mehreren in Kaskade geschalteten Hydrierreaktoren arbeiten, vermeiden einen Teil dieser Nachteile. Es bleibt jedoch erforderlich, den pulverförmigen Katalysator gezielt zu dosieren, umzupumpen und quantitativ vom Reaktionsprodukt abzufiltrieren. Die Katalysatorschlammpumpen unterliegen einem hohen mechanischen Verschleiß. Die quantitative Entfernung des pulverförmigen Katalysators aus dem Reaktionsprodukt ist aufwendig. Ferner ist die Gefahr groß, die Katalysatoraktivität durch die zusätzlichen Operationen verhältnismäßig schnell zu verringern. Es ist daher vorteilhaft, die Reaktion über einen fest angeordneten Katalysator ablaufen zu lassen. Ein solcher Katalysator muß eine hohe Aktivität besitzen, die über einen längeren Zeitraum nicht nachlassen darf, weil häufige Katalysatorwechsel bei Festbettreaktionen ebenfalls aufwendig sind.

Aus EP-A 152 779 ist ein Verfahren zur kontinuierlichen Hydrierung von α-D-Glucopyranosido-1,6-fructose zu einem Gemisch der Zuckeralkohole α-D-Glucopyranosido-1,6-mannit und -sorbit über trägerfreien Formkörpern von Elementen der 8. Nebengruppe des Periodensystems bekannt, wobei diese trägerfreien Formkörper vorzugsweise durch Verpressen und/oder Verkleben von Metallpulvern hergestellt worden sind.

Aus DE-A 4 416 115 ist ein Verfahren zur kontinuierlichen Hydrierung von α-D-Glucopyranosido-1,6-fructose zu einem Gemisch der korrespondierenden Zuckeralkohole über trägerfreien Formkörpern von Elementen der Eisenuntergruppe der VIII. Nebengruppe des Periodensystem mit Elementen der VI. Nebengruppe bekannt. Leider fallen bei diesen Verfahren die beiden Zuckeralkohole nicht in äquimolaren Mengen an, was aus physikalischen Gründen wünschenswert wäre (stets gleicher Erstarrungspunkt der Mischung) und beim Einsatz in pharmazeutischen Verwendungen (vorgeschriebene Festschreibung der Zusammensetzung) notwendig ist, sondern je nach Reaktionstemperatur in unterschiedlichen Mengenverhältnissen. Es bleibt ferner wünschenswert, die stündliche Katalysatorbelastung merklich zu erhöhen und die Katalysatorkosten weiter zu senken. Außerdem ist man immer bestrebt, ein Verfahren bei möglichst niedriger Temperatur durchzuführen, um die Energiekosten zu senken.

Überraschenderweise wurde jetzt gefunden, daß Elemente der IV. und V. Nebengruppe des Periodensystems enthaltende aus billigen Legierungsabfällen stammende Metallpulver von Nickel-, Kobalt- und Eisen- Legierungen nach ihrer Verpressung zu Formkörpern nicht nur erfolgreich die Hydrierung von α-D-Glucopyranosido-1,6-fructose zu einem äquimolaren Gemisch von α-D-Glucopyranosido-1,6-mannit und α-D-Glucopyranosido-1,6-sorbit katalysieren, sondern daß Katalysatoren aus diesen Metall-Legierungen, die um 50 - 70 % preiswerter sind als reine Metalle, auch noch eine erheblich höhere Hydrieraktivität aufweisen, so daß die Hydrierreaktion bei einer bis zu 50°C niedrigeren Reaktionstemperatur gegenüber bisherigen Verfahren durchgeführt werden kann. Dabei können die zum Einsatz kommenden Pulver sogar noch zusätzlich gewisse Verunreinigungen an anderen nicht katalytisch wirkenden Elementen oder Element-Legierungen (z.B. Mangan, Silicium, Aluminium) enthalten, ohne daß die hohe Aktivität gemindert wird.

Gegenstand der Erfindung ist daher ein Verfahren zur Herstellung eines äquimolaren Gemisches von α-D-Glucopyranosido-1,6-mannit und α-D-Glucopyranosido-1,6-sorbit aus α-D-Glucopyranosido-1,6-fructose durch katalytische Hydrierung in wäßriger Lösung mit Wasserstoff unter erhöhtem Druck und bei erhöhter Temperatur, das dadurch gekennzeichnet ist, daß man die Hydrierung kontinuierlich bei einem Wasserstoffdruck von 100 bis 400 bar und einer Temperatur von 20 bis 80°C im Festbettverfahren über als Hydrierungskatalysatoren dienenden trägerfreien Formkörpern mit einer Druckfestigkeit von 20 bis 220 N und einer inneren Oberfläche von 10 bis 95 m²/g von (i) einem oder mehreren Elementen der Eisenuntergruppe der VIII. Nebengruppe des Periodensystems der Elemente (Mendelejew) durchführt, (das) die zusätzlich mit (ii) aktivierend wirkenden Elementen der IV. und/oder V. Nebengruppe des Periodensystems der Elemente (Mendelejew) legiert ist (sind).

Die Eisenuntergruppe der VIII. Nebengruppe des Periodensystem der Elemente (Mendelejew) enthält die Elemente Eisen, Cobalt, und Nickel. Die erfindungsgemäß zu verwendenden trägerfreien Formkörper enthalten eines oder mehrere dieser Metalle einzeln oder legiert, bevorzugt legiert, in Mengen von mindestens 50, vorzugsweise mindestens 60, insbesondere mindestens 70 Gew.-%, bezogen auf das Gesamtgewicht der trägerfreien Formkörper.

Die IV. Nebengruppe des Periodensystem enthält die Elemente Titan, Zirkonium und Hafnium. Die V. Nebengruppe des Periodensystem enthält die Elemente Vanadium, Niob, Tantal. Die erfindungsgemäß zu verwendenden trägerfreien Formkörper enthalten eines oder mehrere dieser Elemente einzeln oder legiert, bevorzugt legiert, mit den Elementen der VIII. Nebengruppe in Mengen von mindestens 1,5, vorzugsweise mindestens 3,0, insbesondere mindestens 6,0 Gew.-%, bezogen auf das Gesamtgewicht der trägerfreien Formkörper; sie enthalten eines oder mehrere dieser Elemente in Mengen von höchstens 30, vorzugsweise höchstens 20 und insbesondere höchstens 15 Gew.-%, bezogen auf das Gesamtgewicht der trägerfreien Formkörper.

Die erfindungsgemäß zu verwendenden trägerfreien Formkörper können darüber hinaus, jeweils bezogen auf das Gesamtgewicht der trägerfreien Formkörper, bis zu 20, vorzugsweise bis zu 18, insbesondere bis zu 15 Gew.-% anderer Elemente einzeln oder legiert enthalten; Beispiele solcher Elemente, die nicht katalytisch wirken müssen, umfassen Aluminium, Silicium und Mangan. Nach einer bevorzugten Ausführungsform enthalten die trägerfreien Formkörper außer den Komponenten (i) und (ii) nicht mehr als 12 Gew.-% Aluminium und nicht mehr als 5 Gew.-% anderer Elemente außer Al.

Die Herstellung der trägerfreien Formkörper kann nach gebräuchlichen Methoden durch Verpressen von Elementpulvern mit Korngrößen bis zu 200 µm auf Tablettier- oder Pelletiermaschinen unter hohem Druck erfolgen, wobei zur Verbesserung des Haftvermögens der Metallpartikel auch Graphit in Mengen von 0,5 - 1,5 Gew.-%, bezogen auf das Gesamtgewicht der den Katalysator bildenden Bestandteile, oder Klebstoffe in ähnlichen Mengen zum Einsatz kommen können. Zur Herstellung können Pulver der vorgesehenen Elemente gemischt und verpreßt werden; es ist jedoch auch möglich, Pulver aus geeigneten Legierungen einzusetzen. Solche Legierungen haben entweder die in Frage kommende Zusammensetzung oder eine in der Nähe liegende, die durch Ergänzungszusätze geeigneter Elemente angepaßt wird. Hierzu können Legierungsabfälle und -reste beliebiger Herkunft verwendet werden. Die Herstellung der trägerfreien Formkörper erfolgt vorzugsweise in einer sauerstofffreien Atmosphäre, um Oberflächenoxidationen zu vermeiden. Am wirksamsten und für die Reaktionsführung am günstigsten sind tablettierte oder pelletierte Formkörper mit Abmessungen von 3 bis 7 mm. Von erheblicher Bedeutung ist die Druckfestigkeit der Formkörper, die erfindungsgemäß bei Werten von 20 bis 220 N, bevorzugt 110 bis 220 N, liegt. Niedrigere Druckfestigkeiten könnten zu Formkörperzerfall bzw. erosivem Anhieb führen, was eine metallische Kontaminierung des Reaktionsproduktes bewirken wurde. Von erheblicher Bedeutung ist weiterhin die innere Oberfläche der Formkörper, die erfindungsgemäß bei Werten von 10 bis 95 m²/g liegt und ausschlaggebend für einen möglichst quantitativen Umsatz der Einsatzstoffe ist. Die verpreßten Formkörper haben makroskopisch glatte Außenflächen.

Die Druckfestigkeit der trägerfreien Formkörper kann nach DIN 50 106 bestimmt werden und wird als Druckfestigkeit auf die gewölbten Preßoberflächen verstanden. Die Überprüfung von trägerfreien Formkörpern auf die anspruchsgemäßen inneren Oberflächen und damit auf Brauchbarkeit für das erfindungsgemäße Verfahren kann nach Methoden durchgeführt werden, die von F. M. Nelsen und F.T. Eggertsen, Analyt. Chem. 30 (1958), 1387 bzw. S. J. Gregg und S. W. Sing, Adsorption, Surface Area and Porosity, London 1967, Kap. 2 und 8, beschrieben worden sind.

Nach dem erfindungsgemäßen Verfahren ist es möglich, das kristallisierte Gemisch der beiden diastereomeren Zuckeralkohole ohne weitere Reinigungsoperationen in einer Reinheit von über 99,5 Gew.-% herzustellen, wobei der Gehalt an nicht umgesetzter α-D-Glucopyranosido-1,6-fructose bei ≤0,1 Gew.-% und die Summe von Sorbit und Mannit unter 0,3 Gew.-% liegen. Die beiden Zuckeralkohole fallen als im wesentlichen äquimolares Gemisch an; dies bedeutet ein Gemisch im Bereich von 49 bis 51 : 51 bis 49 Gew.-%.

Als Ausgangsverbindung für das erfindungsgemäße Verfahren wird kristalline α-D-Glucopyranosido-1,6-fructose eingesetzt. Diese Substanz kann aus Saccharose-Lösung durch enzymatische Umwandlung mit lebenden oder immobilisierten Zellsystemen nach bekannten Methoden (z.B. DE-PS 1 049 800) hergestellt werden. Die α-D-Glucopyranosido-1,6-fructose wird vorteilhafterweise in sauerstofffreiem, entionisiertem Wasser gelöst. Man geht beispielsweise folgendermaßen vor: Aus α-D-Glucopyranosido-1,6-fructose und entionisiertem Trinkwasser wird eine 15- bis 60 gew.-%ige, bevorzugt 40- bis 55 gew.-%ige, wäßrige Lösung hergestellt, deren pH-Wert auf 4,5 - 11,5 bevorzugt 5 - 8,5, eingestellt wird. Kristalline α-D-Glucopyranosido-1,6-fructose zeigt, gelöst in Wasser mit einem pH-Wert von 7, entweder eine neutrale oder, bedingt durch eine möglicherweise durch Cannizarro-Reaktion hervorgerufene spurenweise Gluconsäurebildung, eine schwach saure Reaktion. Die gewünschte pH-Wert-Einstellung kann z.B. durch Zugabe einer möglichst reinen organischen Säure, wie Zitronensäure, Sorbinsäure sowie Zuckersäuren bzw. durch Zugabe von (NH₄)₂-CO₃-Salz zur wäßrigen Lösung erfolgen.

Für den Hydrierprozeß wird ein auf einen Druck von 100 bis 400 bar, bevorzugt 200 bis 300 bar, vorkomprimierter reiner Wasserstoff in der 1-20fachen molaren, bevorzugt 1-10fachen molaren Menge eingesetzt. Die Hydrierung erfolgt kontinuierlich im Festbettverfahren an den als Hydrierkatalysatoren dienenden trägerfreien Formkörpern metallischer Art, indem man die zu hydrierende Lösung entweder mit dem zuvor zugemischten Wasserstoff von unten oder oben kommend über die in einen Hydrierreaktor gefüllten Formkörper strömen läßt (Gleichstromverfahren) oder auch von unten kommend dem von oben einströmenden Wasserstoff entgegenführt oder umgekehrt (Gegenstromverfahren). Der Hydrierreaktor kann entweder ein einzelnes Hochdruckrohr aus Stahl oder einer Stahllegierung sein, das mit den trägerfreien Formkörpern ganz oder teilweise gefüllt wird, wobei auch die Anordnung auf Horden, Drahtkörben o. ä. nützlich sein kann, oder aber ein ummanteltes Hochdruckrohrbündel, dessen Einzelrohre mit den Formkörpern in der genannten Weise gefüllt werden.

Der Hydrierprozeß wird bei einer Temperatur von 20 bis 80°C, bevorzugt 40 bis 70°C, durchgeführt. Niedrigere Temperaturen bedingen höhere Verweilzeiten oder Verzicht auf einen quantitativen Umsatz der α-D-Glucopyranosido-1,6-fructose. Höhere Temperaturen führen zu vermehrter Bildung von Zuckermonoalkoholen (Sorbit bzw. Mannit) sowie zu unkontrollierten Nebenreaktionen (Karamelisierung, hydrierende Crackung), was zu Verfärbungen sowie zur Bildung weiterer unerwünschter Nebenprodukte führen kann. Die stündliche Katalysatorbelastung kann 45 bis 550 g α-D-Glucopyranosido-1,6-fructose/l Katalysator betragen. Bei den geschilderten Reaktionsbedingungen sind ganz unerwartet hohe Katalysatorstandzeiten von 15.000 Stunden und mehr zu erzielen, was zu Katalysatorverbräuchen von ≤0,12 Gew.-%, bezogen auf das Reaktionsprodukt, führt.

Damit liegen die hauptsächlichen technischen Vorteile des erfindungsgemäßen Verfahrens außer in den hohen Ausbeuten an α-D-Glucopyranosido-1,6-mannit und α-D-Glucopyranosido-1,6-sorbit in äquimolaren Mengen und der Reinheit des Gemisches, die keine weiteren Reinigungsprozeduren notwendig macht, besonders in den niedrigen Katalysatorkosten und der niedrigen Reaktionstemperatur.

Die den Reaktor verlassende hydrierte wäßrige Lösung, die die beiden Zuckeralkohole α-D-Glucopyranosido-1,6-mannit und -sorbit im Verhältnis von 1 : 1 enthält, wird nach der Entspannung, bei welcher man den überschüssigen Wasserstoff abfangen und nach erfolgter Komprimierung erneut zum Einsatz bringen kann, filtriert und kann so bereits direkt als Zuckeraustauschstoffmischung für diätetische Zwecke in flüssiger Form zum Einsatz kommen. Das Wasser der Lösung läßt sich aber auch auf bekannte Weise über Sprühtrockner, Trockenwalzen oder durch Gefriertrocknung entfernen. Im Regelfall wird die nach dem Filtrieren erhaltene farblose und glasklare Lösung in einem Fallfilmverdampfer oder einem ähnlich arbeitenden Gerät auf einen Zuckeralkoholgehalt von ca. 80 Gew.-% aufkonzentiert und anschließend in einem Vakuum-Kristallisationsgerät zur vollständigen Kristallisation gebracht. Das Kristallisat läßt sich durch einen nachgeschalteten Mahlprozeß und eventuell Siebung auf eine einheitliche Korngröße bringen. Obwohl das so gewonnene Produkt rieselfähig ist und völlig trocken erscheint, hat es einen Kristallwassergehalt von ca. 5 Gew.-%, was darauf zurückzuführen ist, daß α-D-Glucopyranosido-1,6-mannit im Gegensatz zu α-D-Glucopyranosido-1,6-sorbit mit einem Gehalt von etwa 10 Gew.-% Kristallwasser kristallisiert. Das gewonnene Produkt beginnt bei 95°C zu schmelzen. Eine klare Schmelze bildet sich bei 141°C. Den exakten Schmelzbereich des wasserfreien Substanzgemisches erhält man, wenn man z.B. das wasserhaltige Produkt in einem evakuierbaren Trockengerät bei 110°C und 10 mbar zur Schmelze bringt und aus der Schmelze das Wasser quantitativ verdampfen läßt. Eine so behandelte rekristallisierte Probe zeigt ein Schmelzintervall von 140 bis 143°C.

Die beiden erfindungsgemäß äquimolar herstellbaren Zuckeralkohole sind bezüglich ihres Verwendungsgebietes bekannt. Sie werden als Einzelstoffe wie als Mischung aufgrund ihres angenehmen süßen Geschmacks, der im Gegensatz zu manchen anderen Zuckeralkoholen frei von Bei- oder Nebengeschmack ist, als kalorienarme Zuckerersatzstoffe eingesetzt, die auch für Diabetiker geeignet und weniger kariogen sind als Saccharose.

In seinem Lösungsverhalten in Wasser liegt das äquimolare Gemisch von α-D-Glucopyranosido-1,6-mannit und α-D-Glucopyranosido-1,6-sorbit im Temperaturbereich von 0 - 70°C zwischen dem der Reinsubstanzen. Bei Temperaturen über 70°C übertrifft die Löslichkeit des Gemischs die der Reinsubstanzen, was den Einsatz der Mischung als Süßungsmittel für Getränke und Nahrungsmittel immer dann besonders vorteilhaft erscheinen läßt, wenn die infrage kommenden Stoffe stärker gesüßt werden sollen. Sowohl die Einzelverbindungen, die beispielsweise durch fraktionierte Kristallisation erhältlich sind, als auch das Gemisch zeigen eine Süßkraft, die etwa 45 % der Süßkraft von Saccharose entspricht. Zur Erhöhung der Süßkraft des äquimolren Gemisches von α-D-Glucopyranosido-1,6-mannit und α-D-Glucopyranosido-1,6-sorbit kann die wäßrige Lösung mit künstlichen Süßstoffen, z.B. Cyclohexylsulfamat oder Phenylalanin-asparaginsäuremethylester, versetzt und durch gemeinsame Vakuumkristallisation in kristalliner Form gewonnen werden. Man kann aber auch künstlichen Süßstoffe in fester Form mit Kristallisat vermischen. Das Gemisch von α-D-Glucopyranosido-1,6-mannit und α-D-Glucopyranosido-1,6-sorbit kann auch in flüssiger oder fester Form mit anderen süßschmeckenden Zuckeralkoholen, z.B. Sorbit, Xylit, Mannit und/oder Lactit vermischt werden. Wegen seiner hohen Karamelisierungstemperatur ist das Gemisch besonders gut als süß schmeckender, struktur- und körperbildender Füllstoff für Schokolade, Marzipan, Pralinen und Gebäck geeignet.

### Beispiele (Die Prozentangaben beziehen sich auf das Gewicht)

### Beispiel 1

Ein senkrecht stehendes, wärmeisoliertes Hochdruckrohr aus nichtrostendem Stahl von 45 mm Innendurchmesser und 1 m Länge wurde mit 1,4 l eines durch Tablettierung von Pulver einer Ni/Zr-Legierung mit einem Zr-Gehalt von 19,8 % hergestellten Hydrierungskatalysators gefüllt, der bei einer Zylinderhöhe von 5 mm und einem Durchmesser von 5 mm eine Druckfestigkeit von 109 N und eine innere Oberfläche von 74 m²/g aufwies. Durch dieses Rohr wurden gemeinsam mit der dreifach molaren Menge von unter einem Druck von 300 bar stehendem hochreinem Wasserstoff stündlich 300 ml einer 40 %igen Lösung von α-D-Glucopyranosido-1,6-fructose in entionisiertem Trinkwasser, die auf einen pH-Wert von 6,0 eingestellt worden war, kontinuierlich von unten nach oben aufsteigend gepumpt. Wäßrige Lösung und Wasserstoff wurden durch einen Wärmeaustauscher geführt und so hoch erhitzt, daß sie in das Hochdruckrohr mit einer Temperatur von 50°C eintraten. Das das Hochdruckrohr verlassende Gemisch von wäßriger Lösung und überschüssigem Wasserstoff wurde über einen Kühler in einen Abscheider geführt, von wo der Wasserstoff nach Einsatz der verbrauchten Menge wieder zusammen mit noch nicht hydrierter Lösung in den Vorwärmer und von da in das Hochdruckrohr gepumpt wurde. Die klare Lösung wurde entspannt, über ein Feinfilter filtriert, in einem Fallfilmverdampfer auf einen Zuckeralkoholgehalt von ca. 80 % aufkonzentriert und anschließend in einem Vakuumkristaller zur vollständigen Kristallisation gebracht. Das erhaltene feine Kristallpulver bestand aus einem Gemisch aus α-D-Glucopyranosido-1,6-mannit und α-D-Glucopyranosido-1,6-sorbit im Verhältnis 1:1. Der Wassergehalt lag bei 5 %. Das Gemisch der beiden stereoisomeren Zuckeralkohole war ansonsten hochrein (Reinheitsgrad: 99,6 %). Der Gehalt an nicht hydrierter α-D-Glucopyranosido-1,6-fructose lag bei ≤0,1 %. Der Gehalt an Sorbit lag bei ≤0,1 %. Mannit konnte nicht nachgewiesen werden. Der Katalysator war auch nach einer Laufzeit von 3822 Stunden unverändert wirksam.

### Beispiel 2

Durch ein Hochdruckrohr wie im Beispiel 1, jedoch aus Hochdruckstahl N 9, wurden bei einer Temperatur von 65°C und einem Wasserstoffdruck von 200 bar im umgekehrten Wasserstofffluß, wie im Beispiel 1 beschrieben, stündlich 600 ml einer 40 %igen wäßrigen Lösung von α-D-Glucopyranosido-1,6-fructose hydriert, die einen pH-Wert von 8,5 aufwies. Der Katalysator war durch Tablettierung von Pulver einer Ni/Zr-Legierung mit einem Zr-Gehalt von 14,9 % und einem Al-Gehalt von 10,5 % hergestellt worden. Die Tabletten hatten bei einer Zylinderhöhe von 5 mm und einem Durchmesser von 5 mm eine Druckfestigkeit von 75 N und eine innere Oberfläche von 81 m²/g. Nach einer Laufzeit von 1216 Stunden mit unverminderter Wirksamkeit des Katalysators lag der Gehalt an α-D-Glucopyranosido-1,6-mannit und α-D-Glucopyranosido-1,6-sorbit des in einem Rotationsverdampfer zur Trocknung eingedampften Reaktionsgemisches bei 99,6 %. Der Gehalt an nicht hydrierter α-D-Glucopyranosido-1,6-fructose lag bei < 0,1 %. Der Gehalt an Sorbit lag bei 0,1 %. Der Gehalt an Mannit betrug 0,02 %.

### Beispiel 3

In einem Hochdruckrohr wie im Beispiel 1 wurde bei einer Temperatur von 45°C und einem Wasserstoffdruck von 300 bar in gleicher Weise wie in Beispiel 1 stündlich eine gleichgroße Menge einer 55 %igen wäßrigen Lösung von α-D-Glucopyranosido-1,6-fructose hydriert, die einen pH-Wert von 8,5 aufwies. Der Katalysator war durch Tablettierung einer pulverisierten Ni/Fe/Zr-Legierung hergestellt worden. Die Legierung enthielt einen Fe-Anteil in Ni von 5 % sowie einen Zr-Anteil von 10,9 %. Die Tabletten hatten bei einer Zylinderhöhe von 3 mm und einem Durchmesser von 3 mm eine Druckfestigkeit von 103 N und eine Oberfläche von 95 m²/g. Das in einem Vakuumkristaller gewonnene 1:1 - Gemisch von α-D-Glucopyranosido-1,6-mannit und α-D-Glucopyranosido-1,6-sorbit hatte einen Reinheitsgrad von ≥99,5 %. Der Gehalt an nicht umgesetzter α-D-Glucopyranosido-1,6-fructose lag bei < 0,1 %. Der Sorbit-Gehalt betrug 0,1 %. Der Mannitanteil betrug 0,02 %. Der Katalysator war nach einer Laufzeit von 1206 Stunden noch unverändert wirksam.

### Beispiel 4

In einem Hochdruckrohr wie in Beispiel 1 wurde bei einer Temperatur von 55°C und einem Wasserstoffdruck von 200 bar in gleicher Weise wie in Beispiel 1 eine ebenso große Menge einer 45 %igen wäßrigen Lösung von α-D-Glucopyranosido-1,6-fructose hydriert, die einen pH-Wert von 6,5 aufwies. Der Katalysator war durch Tablettierung einer pulverisierten Ni/Zr-Legierung und einer pulverisierten Ni/Ti-Legierung hergestellt worden und hatte einen Zr-Gehalt von 12,1 % und einen Ti-Gehalt von 5,8 %. Die Tabletten hatten bei einer Zylinderhöhe von 5 mm und einem Durchmesser von 5 mm eine Druckfestigkeit von 103 N und eine innere Oberfläche von 81 m²/g. Das in einem Vakuum-Drehrohr gewonnene Gemisch von α-D-Glucopyranosido-1,6-mannit und α-D-Glucopyranosido-1,6-sorbit hatte einen Reinheitsgrad von > 99,5 %. Der Gehalt an nicht umgesetzter α-D-Glucopyranosido-1,6-fructose lag bei < 0,1 %. Der Sorbit-Gehalt betrug 0,1 %. Mannit ließ sich nicht nachweisen. Der Katalysator war nach einer Laufzeit von 4689 Stunden noch unvermindert wirksam.

### Beispiel 5

In einem Hochdruckrohr wie in Beispiel 1 wurden bei einer Temperatur von 65°C und einem Wasserstoffdruck von 300 bar in gleicher Weise wie in Beispiel 2 eine ebenso große Menge einer 40 %igen wäßrigen Lösung von α-D-Glucopyranosido-1,6-fructose hydriert, die einen pH-Wert von 6,5 aufwies. Der Katalysator war durch Tablettierung einer pulverisierten Ni/Zr/V/Al-Legierung mit einem Zr-Gehalt von 14,9 %, einem V-Gehalt von 6,4 % und einen Al-Gehalt von 10,4 % hergestellt worden. Die Tabletten hatten bei einer Zylinderhöhe von 5 mm und einem Durchmesser von 5 mm eine Druckfestigkeit von 95 N und eine innere Oberfläche von 78 m²/g. Das in einem Vakuumkristaller gewonnene 1:1 - Gemisch von α-D-Glucopyranosido-1,6-mannit und α-D-Glucopyranosido-1,6-sorbit hatte einen Reinheitsgrad von > 99,5 %. Der Gehalt an nicht umgesetzter α-D-Glucopyranosido-1,6-fructose lag bei 0,1 %. Der Sorbit-Gehalt betrug 0,1 %. Der Mannitanteil betrug 0,02 %. Der Katalysator war nach einer Laufzeit von 1618 Stunden noch unvermindert wirksam.

### Beispiel 6

In einem Hochdruckrohr wie in Beispiel 1 wurde bei einer Temperatur von 55°C und einem Wasserstoffdruck von 150 bar in gleicher Weise wie in Beispiel 1 eine ebenso große Menge einer 45 %igen wäßrigen Lösung von α-D-Glucopyranosido-1,6-fructose hydriert, die einen pH-Wert von 6,5 aufwies. Der Katalysator war durch Tablettierung einer pulverisierten Ni/V-Legierung gewonnen, die auch Al enthielt. Die Legierung besaß einen V-Gehalt von 6,1 % und einen Al-Gehalt von 9,4 %. Die Tabletten hatten bei einer Zylinderhöhe von 5 mm und einem Durchmesser von 5 mm eine Druckfestigkeit von 95 N und eine innere Oberfläche von 68 m²/g. Das in einem Vakuum-Drehrohr gewonnene 1:1 - Gemisch von α-D-Glucopyranosido-1,6-mannit und α-D-Glucopyranosido-1,6-sorbit hatte einen Reinheitsgrad von > 99,5 %. Der Gehalt an nicht umgesetzter α-D-Glucopyranosido-1,6-fructose lag bei 0,1 %. Der Sorbit-Gehalt betrug 0,05 %. Mannit ließ sich nicht nachweisen. Der Katalysator war nach einer Laufzeit von 918 Stunden noch unvermindert wirksam.

## Patentansprüche

1. Verfahren zur Herstellung eines äquimolaren Gemisches von α-D-Glucopyranosido-1,6-mannit und α-D-Glucopyranosido-1,6-sorbit aus α-D-Glucopyranosido-1,6-fructose durch katalytische Hydrierung in wäßriger Lösung mit Wasserstoff unter erhöhtem Druck und bei erhöhter Temperatur, dadurch gekennzeichnet, daß man die Hydrierung kontinuierlich bei einem Wasserstoffdruck von 100 bis 400 bar und einer Temperatur von 20 bis 80°C im Festbettverfahren über als Hydrierungskatalysatoren dienenden trägerfreien Formkörpern mit einer Druckfestigkeit von 20 bis 220 N und einer inneren Oberfläche von 10 bis 95 m²/g von (i) einem oder mehreren Elementen der Eisenuntergruppe der VIII. Nebengruppe des Periodensystem der Elemente (Mendelejew) durchführt, das (die) zusätzlich mit (ii) aktivierend wirkenden Elementen der IV. und/oder V. Nebengruppe des Periodensystems der Elemente (Mendelejew) legiert ist (sind).

2. Verfahren nach Anspruch 1, dadurch gekennzeichnet, daß die Formkörper einen Gewichtsanteil an einem oder mehreren Elementen der Eisenuntergruppe von mindestens 50 %, bevorzugt mindestens 60 %, besonders bevorzugt mindestens 70 % haben.

3. Verfahren nach Anspruch 1, dadurch gekennzeichnet, daß die Formkörper einen Gewichtsanteil an einem oder mehreren Elementen der IV. oder V. Nebengruppe von mindestens 1,5 %, bevorzugt mindestens 3,0 %, besonders bevorzugt mindestens 6 % haben.

4. Verfahren nach Anspruch 1, dadurch gekennzeichnet, daß die Formkörper Abmessungen von 3 bis 7 mm besitzen.

5. Verfahren nach Anspruch 1, dadurch gekennzeichnet, daß die Formkörper neben Elementen der Eisenuntergruppe und der IV. bzw. V. Nebengruppe weitere Elemente aus der Gruppe Aluminium, Silicium und Mangan mit einem Gewichtsanteil von bis zu 20 %, bevorzugt bis zu 18 %, besonders bevorzugt bis zu 15 % haben.

6. Verfahren nach Anspruch 5, dadurch gekennzeichnet, daß die Formkörper einen Gewichtsanteil von nicht mehr als 12 % Aluminium und nicht mehr als 5 % anderer Elemente außer Al haben.

7. Verfahren nach Anspruch 1, dadurch gekennzeichnet, daß die Formkörper eine Druckfestigkeit von 110-220 N haben.

8. Verfahren nach Anspruch 1, dadurch gekennzeichnet, daß man die Hydrierung der α-D-Glucopyranosido-1,6-fructose in 15- bis 60 gew.%iger wäßriger Lösung, bevorzugt in 40- bis 55 gew.-%iger Lösung, bei einem pH-Wert von 4,5 bis 11,5, bevorzugt von 5 bis 8,5 durchführt.

9. Verfahren nach Anspruch 1, dadurch gekennzeichnet, daß die Hydrierung bei einem H₂-Druck von 200 bis 300 bar durchgeführt wird.

10. Verfahren nach Anspruch 1, dadurch gekennzeichnet, daß die Hydrierung bei 40 bis 70°C durchgeführt wird.
